# DEMANDE DE BREVET EUROPEEN

(11) **EP 4 674 836 A2**
(43) Date de publication de la demande: **07.01.2026**
(21) Numéro de dépôt: 25217063.4
(22) Date de dépôt: 16.12.2021
(51) Int. Cl.: C07C 321/04

(54) **PROCEDE DE PREPARATION DE METHYLMERCAPTAN AVEC TRAITEMENT DES REJETS GAZEUX**

(30) Priorité: 17.12.2020 FR 2013438
(62) Demande divisionnaire de: 21851836.3
(71) Demandeur: ARKEMA France, 92800 Puteaux (FR)
(72) Inventeur: FREMY, Georges, 64170 LACQ (FR); RAYMOND, Jean-Michel, 40300 CAUNEILLE (FR); LAMANT, Eric, 64170 LACQ (FR)
(74) Mandataire: Arkema Patent

(57) **Abrégé**

La présente invention concerne un procédé de production de méthylmercaptan comprenant les étapes suivantes :
A) on fait réagir du méthanol avec de l'hydrogène sulfuré pour former un flux (M), de préférence à l'état gazeux, comprenant du méthylmercaptan, de l'H₂S n'ayant pas réagi et éventuellement des sous-produits soufrés ;
B) éventuellement, on condense ledit flux (M) ;
C) on effectue au moins une étape de purification dudit flux (M) pour obtenir un flux enrichi en méthylmercaptan ;
D) on récupère les évents gazeux issus de ladite au moins une étape de purification, lesdits évents gazeux comprenant au moins un composé soufré, de préférence l'H₂S ;
E) on réalise une extraction gaz-liquide dudit au moins un composé soufré, de préférence l'H₂S, par du méthanol liquide de façon à obtenir un méthanol liquide enrichi en composé(s) soufré(s), de préférence en H₂S ; et
F) éventuellement, on utilise ledit méthanol enrichi en tant que réactif pour la réaction de l'étape A).

## Description

La présente invention concerne un procédé de production de méthylmercaptan intégrant le traitement des rejets gazeux. La présente invention concerne également un procédé de traitement des rejets gazeux d'une unité de production de méthylmercaptan.

Les mercaptans présentent un grand intérêt industriel et sont aujourd'hui très largement utilisés par les industries chimiques, notamment comme matières premières pour la synthèse de molécules organiques plus complexes. Par exemple, le méthylmercaptan (CH₃SH ou MeSH) est utilisé comme matière première dans la synthèse de la méthionine, acide aminé essentiel pour l'alimentation animale. Le méthylmercaptan est également utilisé dans la synthèse de disulfures de dialkyles, en particulier dans la synthèse du disulfure de diméthyle (DMDS), additif de sulfuration de catalyseurs d'hydrotraitement de coupes pétrolières, entre autres applications.

La synthèse industrielle du méthylmercaptan peut se faire selon « la voie méthanol », à partir de méthanol et d'hydrogène sulfuré selon la réaction (1) suivante :

CH₃OH + H₂S - > CH₃SH + H₂O (1)

La réaction (1) est généralement réalisée en phase gazeuse avec de l'H₂S en excès. Aussi, au niveau industriel, des rejets gazeux (également appelés évents gazeux) peuvent être émis tout au long du procédé de production. Ces rejets sont habituellement traités par incinération. Or, ils comprennent une très forte concentration en H₂S dont la combustion lors de l'incinération va conduire à de fortes émissions de dioxyde de soufre dans l'atmosphère (le SO₂ est un gaz polluant et irritant, qui peut être responsable de pluies acides). De plus, cette perte d'H₂S augmente les coûts variables de production du méthylmercaptan et entraine une diminution de la productivité.

Il existe donc un besoin pour un procédé de production de méthylmercaptan qui soit plus respectueux de l'environnement et plus économique. Il existe également un besoin pour diminuer les rejets gazeux, et notamment le dioxyde de soufre, émis lors de la production de méthylmercaptan.

Un objectif de la présente invention est de fournir un procédé de préparation de méthylmercaptan qui permette une gestion améliorée des rejets gazeux, et notamment qui soit plus respectueux de l'environnement.

Un objectif de la présente invention est de diminuer les rejets gazeux, et notamment la quantité de dioxyde de soufre, émis lors de la production de méthylmercaptan. Un autre objectif de la présente invention est de fournir un procédé de préparation de méthylmercaptan qui soit plus économique.

Un autre objectif de la présente invention est de fournir un procédé et/ou un dispositif de traitement des rejets gazeux qui soit(soient) facilement intégrable(s) dans une unité de production de méthylmercaptan, notamment par voie méthanol.

La présente invention répond en tout ou partie aux objectifs mentionnés ci-dessus. Les présents inventeurs ont découvert de façon surprenante que les rejets gazeux peuvent être récupérés et traités par une extraction gaz-liquide. L'extraction gaz(évents)-liquide(méthanol) selon l'invention permet notamment de faire passer les composés soufrés contenus dans lesdits évents gazeux dans le méthanol liquide. Par « composés soufrés », on entend des composés qui comprennent au moins un atome de soufre, de préférence 1 ou 2 atomes de soufre. En particulier, par « composés soufrés » on entend l'H₂S, le méthylmercaptan et les sous-produits soufrés tels que le diméthylsulfure (DMS) et le diméthyldisulfure (DMDS).

Ainsi, l'extraction gaz-liquide selon l'invention permet de récupérer au moins un composé soufré, de préférence l'H₂S, dans le méthanol. En particulier, l'extraction gaz-liquide selon l'invention permet de récupérer l'H₂S et le méthylmercaptan dans le méthanol.

De façon très avantageuse, ledit méthanol enrichi en composé(s) soufré(s) peut être utilisé, de préférence directement utilisé (par exemple sans étape de purification), en tant que réactif pour former du méthylmercaptan. Ainsi, la présente invention permet de réintroduire dans le procédé de production l'H₂S et éventuellement le méthylmercaptan des évents qui étaient jusqu'alors incinérés.

De plus, ladite extraction laisse passer les composés inertes (i.e. les composés inertes ne passent quasiment pas des évents dans le méthanol mais restent dans la phase gazeuse). Ceci évite l'accumulation de ces derniers dans les installations et donc les bouchages et les problèmes de sécurité en découlant. En particulier, l'hydrogène (H₂), ne passe pas dans le méthanol, ce qui permet d'éviter des réactions indésirables de méthanation dans le réacteur lorsque le méthanol enrichi est utilisé en tant que réactif dans la production de méthylmercaptan. Ces réactions sont notamment les suivantes :

CH₃OH + H₂ -> CH₄ + H₂O et CH₃SH +H₂ -> CH₄ + H₂S.

Il est entendu que l'extraction selon l'invention ne vise pas à récupérer les composés soufrés, et en particulier l'H₂S n'ayant pas réagi, en sortie directe de réacteur pour la production de méthylmercaptan à partir de méthanol et d'H₂S. L'extraction selon l'invention ne vise pas non plus la récupération ou le recyclage de la majeure partie de l'H₂S n'ayant pas réagi. Le procédé selon l'invention vise à récupérer les composés soufrés présents dans les évents gazeux, habituellement incinérés et responsables du relargage du dioxyde de soufre.

Ainsi, le procédé selon l'invention n'implique notamment pas une quantité de méthanol pour ladite extraction supérieure à la quantité de méthanol nécessaire pour la synthèse du méthylmercaptan. Au contraire, l'extraction selon l'invention est facilement intégrable à une unité de production de méthylmercaptan car elle traite uniquement les évents gazeux. Elle consomme donc peu d'énergie et utilise un dispositif simple.

Ladite extraction permet également de diminuer la quantité des évents à traiter par incinération et permet de diminuer fortement le relargage de dioxyde de soufre dans l'atmosphère.

Le procédé de production de méthylmercaptan selon l'invention est donc plus économique, présente une meilleure productivité, tout en étant plus respectueux de l'environnement.

Ainsi, la présente invention concerne un procédé de production de méthylmercaptan comprenant les étapes suivantes :
A) on fait réagir du méthanol avec de l'hydrogène sulfuré pour former un flux (M), de préférence à l'état gazeux, comprenant du méthylmercaptan, de l'H₂S n'ayant pas réagi et éventuellement des sous-produits soufrés ;
B) éventuellement, on condense ledit flux (M) ;
C) on effectue au moins une étape de purification dudit flux (M) pour obtenir un flux enrichi en méthylmercaptan ;
D) on récupère les évents gazeux issus de ladite au moins une étape de purification, lesdits évents gazeux comprenant au moins un composé soufré, de préférence l'H₂S ;
E) on réalise une extraction gaz-liquide dudit au moins un composé soufré (présent dans lesdits évents), de préférence l'H₂S, par du méthanol liquide de façon à obtenir un méthanol liquide enrichi en composé(s) soufré(s), de préférence en H₂S ; et
F) éventuellement, on utilise ledit méthanol enrichi en tant que réactif pour la réaction de l'étape A).

En particulier, les évents gazeux comprennent de l'H₂S, du méthylmercaptan et éventuellement des sous-produits soufrés de façon à obtenir suite à l'étape E) un méthanol enrichi en H₂S, en méthylmercaptan et éventuellement en sous-produits soufrés.

On entend par « méthanol enrichi », le méthanol obtenu après l'extraction gaz-liquide telle que selon l'invention, notamment obtenu après l'étape E).

En particulier, le méthanol enrichi est une composition comprenant du méthanol et au moins un composé soufré, de préférence comprenant du méthanol et de l'H₂S, éventuellement du méthylmercaptan et éventuellement des sous-produits soufrés tels que le DMS et le DMDS. En particulier, ledit méthanol enrichi comprend entre 0,1% et 20% en poids d'H₂S, de préférence entre 1% et 10%, plus préférentiellement entre 1% et 5% en poids d'H₂S, par rapport au poids total du méthanol enrichi.

Plus particulièrement, on entend par « méthanol enrichi », une composition comprenant :
- du méthanol, de préférence au moins 50% en poids de méthanol, plus préférentiellement au moins 80% en poids, encore préférentiellement au moins 90% en poids de méthanol, par rapport au poids total de la composition ;
- de l'H₂S, de préférence entre 0,1% et 20% en poids d'H₂S, plus préférentiellement entre 1% et 10%, encore plus préférentiellement entre 1% et 5% en poids d'H₂S, par rapport au poids total de la composition ;
- éventuellement du méthylmercaptan ;
- éventuellement des sous-produits soufrés, de préférence le diméthylsulfure et le diméthyldisulfure ;
- éventuellement de l'eau ; et
- éventuellement des composés inertes.

Ladite composition peut ainsi comprendre :
- entre 90 et 99% en poids de méthanol ;
- entre 0,1 et 10%, de préférence entre 0,1 et 5%, en poids d'H₂S ; et
- entre 0,1 et 5% en poids de méthylmercaptan,
par rapport au poids total de la composition.

Par « évent ou rejet gazeux », on entend une phase gazeuse comprenant au moins un composé soufré tel que défini ci-dessus et notamment récupérée suite à au moins une étape de purification du flux (M). Lesdits évents gazeux peuvent comprendre, voire être constitués de, l'H₂S, du méthylmercaptan, des composés inertes, éventuellement de l'eau et des sous-produits soufrés. Ils comprennent en particulier au moins 50%, de préférence au moins 60%, voire au moins 70% en poids d'H₂S par rapport au poids total des évents. Ils peuvent comprendre :
- entre 50% et 90%, de préférence entre 60% et 80%, en poids d'H₂S ;
- entre 5% et 25%, de préférence entre 10% et 20%, en poids de méthylmercaptan ;
- entre 1% et 15% d'inertes ; de préférence entre 5% et 15%, d'inertes ;
par rapport au poids total des évents gazeux, et éventuellement de l'eau et des sous-produits soufrés à l'état de traces.

En particulier, les évents gazeux tels que selon l'invention sont normalement considérés comme des déchets ou des rejets et sont habituellement envoyés à l'incinérateur.

En particulier, les évents gazeux au sens de la présente invention ne sont pas récupérés directement en sortie du réacteur où se déroule l'étape A).

Lesdits évents peuvent être issus d'un décanteur et/ou d'une purge, de préférence d'une purge d'un flux gazeux.

On entend par « inertes » ou « composés inertes », des composés qui ne sont pas chimiquement actifs lors de la préparation du méthylmercaptan à partir de méthanol et d'H₂S. On peut citer comme composés inertes CH₄, CO, CO₂, H₂ et N₂.

On entend par « traces » d'un composé, une quantité comprise entre 0 et 10000 ppm, de préférence entre 0 et 1000 ppm.

On entend notamment par « étape de purification du méthylmercaptan », une étape permettant d'obtenir un flux enrichi en méthylmercaptan. On entend notamment par « flux enrichi en méthylmercaptan » un flux qui comprend un pourcentage en poids de méthylmercaptan (par rapport au poids total dudit flux) supérieur au pourcentage en poids de méthylmercaptan par rapport au poids total dudit flux avant ladite étape de purification.

Selon la présente invention, l'unité ppm (partie par million) fait référence à une fraction massique.

### Etape A) - Réaction :

Lors de l'étape A), on fait réagir du méthanol avec de l'hydrogène sulfuré pour former un flux (M), de préférence à l'état gazeux, comprenant du méthylmercaptan, de l'H₂S n'ayant pas réagi et éventuellement des sous-produits soufrés. Le flux (M) peut contenir également de l'eau et du méthanol n'ayant pas réagi.

Préalablement à l'étape A), on peut préparer un flux gazeux des réactifs H₂S et méthanol comme suit.

Du méthanol liquide est injecté dans de l'H₂S gazeux. Cette injection permet de vaporiser partiellement ou entièrement le méthanol. Le mélange d'H₂S et de méthanol peut ensuite être entièrement vaporisé si nécessaire de façon à obtenir un flux totalement gazeux.

Ainsi, un flux gazeux d'H₂S et de méthanol, de préférence préparé tel que ci-dessus, ou de façon séparée le méthanol et l'H₂S, chacun sous forme gazeuse, sont introduits dans un réacteur.

Ledit réacteur peut être isotherme ou adiabatique, à plaques, multitubulaire ou à lit fixe. On choisit de préférence un réacteur adiabatique.

La température de réaction peut être comprise entre 200° et 500°C, de préférence entre 200°C et 400°C. De préférence, la température de réaction est comprise entre 200°C et 360°C. Au-dessus de cette température, le catalyseur peut être physiquement endommagé (par frittage et cokage notamment).

La pression peut être comprise entre 1 et 40 bars.

Le rapport molaire H₂S/méthanol peut être compris entre 1 et 50, de préférence entre 1 et 25. L'H₂S est de préférence en excès par rapport au méthanol.

Le réacteur peut contenir un catalyseur pour la réaction de formation du méthylmercaptan, de préférence en phase gaz. Parmi les catalyseurs pouvant être utilisés, on peut citer :
- les catalyseurs à base d'alumine ;
- le dioxyde de thorium ThO₂, de préférence déposé sur un support silicaté ;
- les catalyseurs à base de sulfure de cadmium, de préférence sur un support alumine ;
- les catalyseurs à base des oxydes suivants : MgO, ZrO₂, TiO₂ rutile (R) et anatase (A), CeO₂, et γ-Al₂O₃ ;
- les catalyseurs à base d'oxydes de métaux, de préférence dopés par des métaux alcalins (Li, Na, K, Rb, Cs) et éventuellement supportés sur SiO₂, Al₂O₃ ou Nb₂O₅ ;
- les catalyseurs à base de carbonates de métaux alcalins ;
- les catalyseurs à base de sels de métaux alcalins avec certains acides de métaux de transitions (Cr, Mo, W, Ni), imprégnés sur l'alumine gamma ou autres oxydes de métaux ;
- le tungstate de potassium sur alumine K₂WO₄/Al₂P₃.

On obtient ainsi un flux (M) comprenant du méthylmercaptan, de l'H₂S n'ayant pas réagi et éventuellement des sous-produits soufrés.

### Etape B) - Condensation :

On peut éventuellement condenser le flux (M) issu de l'étape A) à l'aide toute technique classique, de préférence à l'aide d'un ou plusieurs condenseur(s) ou économiseur(s). Lors de la condensation, le flux (M) est notamment refroidi aussi bas que possible, pour maximiser l'élimination de l'eau, mais doit être maintenu strictement au-dessus de 16°C pour éviter la formation d'hydrates solides de méthylmercaptan. De préférence, le flux (M) est condensé à une température comprise entre 20°C et 70°C, par exemple entre 30°C et 60°C.

### Etape C) - Purification et Etape D) - Récupération des évents :

De préférence, lors de l'étape C), ladite au moins une étape de purification correspond à au moins une étape de séparation de phases, de préférence par décantation, et/ou à au moins une étape de distillation. L'étape C) peut notamment correspondre à une ou plusieurs étape(s) de séparation de phases, par exemple une ou deux étapes de décantation, et/ou une ou plusieurs étape(s) de distillation, par exemple une ou deux étapes de distillation.

De préférence, les évents gazeux sont récupérés suite à au moins une étape de séparation de phases, notamment par décantation, et/ou en procédant à au moins une purge, de préférence une purge d'un flux gazeux (par exemple d'un flux gazeux comprenant au moins un composé soufré). Ladite purge peut être effectuée après une étape de séparation de phases, sur le flux gazeux obtenu (par exemple sur un flux gazeux comprenant au moins un composé soufré). En particulier, l'étape de décantation permet de séparer un flux aqueux d'un flux organique comprenant du méthylmercaptan et éventuellement un flux gazeux comprenant au moins un composé soufré ; lesdits évents émis lors de la décantation sont alors récupérés. **Il** peut s'agir d'évents récupérés du décanteur ou d'évents récupérés dans l'une des phases séparées par exemple par stripping par gaz inerte ou par stripping thermique. On peut ainsi séparer, de préférence par décantation, à partir du flux (M) :
- un flux gazeux comprenant de l'hydrogène sulfuré n'ayant pas réagi, ledit flux étant purgé de façon à récupérer un évent gazeux ; et
- un flux comprenant du méthylmercaptan, de préférence à l'état liquide.

De préférence, l'étape C) permet par une ou plusieurs étape(s) de purification d'éliminer du flux (M) l'H₂S n'ayant pas réagi et/ou les sous-produits soufrés et/ou l'eau. En particulier, suite à l'étape C), on obtient un flux enrichi en méthylmercaptan. En particulier, on obtient et on récupère selon l'invention les évents gazeux E1 ou E1' et/ou E2 et/ou E3 tels que définis-dessous. On peut ou non les combiner avant de réaliser l'extraction gaz-liquide.

L'étape de purification C) peut être effectuée par toute technique classique et en particulier selon les étapes c1) à c4) telles que décrites ci-dessous.

Ainsi, selon un mode de réalisation, l'étape C) comprend les étapes de purification suivantes :
c1) on sépare, de préférence par décantation, à partir du flux (M) :
   - un flux gazeux (N) comprenant de l'hydrogène sulfuré n'ayant pas réagi, ledit flux (N) étant purgé de façon à récupérer un évent gazeux E1 ;
   - un flux aqueux (O) ; et
   - un flux (P) comprenant du méthylmercaptan, de l'hydrogène sulfuré n'ayant pas réagi, de l'eau et des sous-produits soufrés ;
c2) on effectue une distillation du flux (P) de façon à obtenir :
   - un flux (R) comprenant de l'hydrogène sulfuré, de préférence en tête de colonne ; et
   - un flux (S) comprenant du méthylmercaptan, de l'eau et des sous-produits soufrés, de préférence en fond de colonne ;
c3) on effectue une distillation du flux (S) de façon à obtenir :
   - un flux (T) comprenant du méthylmercaptan et de l'eau, de préférence en tête de colonne ; et
   - un flux (U) comprenant les sous-produits soufrés, de préférence en fond de colonne ;
c4) on effectue une séparation du méthylmercaptan et de l'eau, de préférence par décantation, de façon à obtenir :
   - un flux (V) comprenant du méthylmercaptan et de l'eau ;
   - un flux (W) comprenant de l'eau ; et
   - un évent gazeux E2.

Les évents E1 et/ou E2 peuvent être envoyés à l'absorbeur méthanol (défini ci-après).

Lesdits flux (M) et/ou (P) et/ou (S) et/ou (T) et/ou (V) peuvent éventuellement comprendre du méthanol n'ayant pas réagi et de l'eau, le méthanol étant de préférence à l'état de traces.

### Etape c1 - Séparation :

Lors de l'étape c1) de séparation, de préférence par décantation, on obtient :
- un flux gazeux (N) comprenant de l'hydrogène sulfuré n'ayant pas réagi ;
- un flux aqueux (O) ; et
- un flux (P) comprenant du méthylmercaptan, de l'eau, de l'hydrogène sulfuré n'ayant pas réagi et des sous-produits soufrés.

De préférence, le flux (M) est séparé à une température comprise entre 20°C et 70°C, de préférence, entre 30°C et 60°C. La pression peut être comprise entre 1 et 40 bars absolus.

Le flux (P) obtenu peut notamment être à l'état gazeux ou à l'état liquide. Lorsque le flux (P) est à l'état gazeux, les flux (N) et (P) peuvent être combinés.

En particulier, le flux aqueux (O), de préférence à l'état liquide, comprend au moins 50%, de préférence au moins 70%, plus préférentiellement au moins 90% en poids d'eau, par rapport au poids total de l'eau présente dans le flux (M). Le flux aqueux (O) peut ensuite être envoyé vers un dégazeur. Le flux aqueux dégazé peut ensuite être envoyé au traitement des eaux usées.

Le flux gazeux (N) peut être recyclé à l'alimentation du réacteur de l'étape A) et/ou une purge de ce flux (N) peut être réalisée de façon à éviter l'accumulation d'inertes et/ou d'impuretés. On peut citer par exemple en tant qu'inertes et/ou impuretés : le méthane, CO, CO₂, H₂ et N₂. Le flux gazeux résultant de cette purge est appelé Events E1. Lorsque les flux (N) et (P) sont combinés, le même type de purge peut être effectuée de façon à obtenir un flux gazeux appelé Events E1'. Les évents E1 ou E1' peuvent être envoyés à l'absorbeur méthanol.

### Etape c2 - Elimination de l'H₂S par distillation :

On effectue ensuite une distillation du flux (P) de façon à obtenir :
- un flux (R) comprenant de l'hydrogène sulfuré, de préférence en tête de colonne ; et
- un flux (S) comprenant du méthylmercaptan, de l'eau et des sous-produits soufrés, de préférence en fond de colonne ;

Lors de la distillation, la pression peut être comprise entre 1 et 40 bar absolus et/ou la température peut être comprise entre -60°C et + 60°C en tête de colonne, et entre +20°C et +200°C en fond de colonne.

Le flux (R) comprenant l'H₂S peut être récupéré en tête de colonne, et éventuellement recyclé vers l'alimentation du réacteur de l'étape A).

En particulier, ladite distillation de l'étape c2) permet d'éliminer l'H₂S restant dans le flux (P) (il est entendu que des traces d'H₂S peuvent rester dans le flux (S)).

### Etape c3 - Elimination des sous-produits soufrés par distillation :

On effectue une distillation du flux (S) de façon à obtenir :
- un flux (T) comprenant du méthylmercaptan et de l'eau, de préférence en tête de colonne ; et
- un flux (U) comprenant les sous-produits soufrés, de préférence en fond de colonne.

Lors de la distillation, la pression peut être comprise entre 1 et 40 bar absolus et/ou la température peut être comprise entre +20°C et + 100°C en tête de colonne, et entre +40°C et +200°C en fond de colonne.

En particulier, ladite distillation de l'étape c3) permet d'éliminer les sous-produits soufrés restant dans le flux (S) (il est entendu que des traces des sous-produits soufrés peuvent rester dans le flux (T)).

### Etape c4 - Séparation du méthylmercaptan et de l'eau :

Préalablement à l'étape c4), le flux (T) peut être refroidi aussi bas que possible, pour maximiser l'élimination de l'eau, mais doit être maintenu strictement au-dessus de 16°C pour éviter la formation d'hydrates solides de méthylmercaptan. De préférence, le flux (T) est refroidi à une température comprise entre 20°C et 70°C, par exemple entre 30°C et 60°C.

Ce refroidissement permet de maximiser la séparation de l'eau lors de l'étape c4), tout en maintenant une température strictement supérieure à 16°C pour éviter la formation d'hydrates solides de méthylmercaptan.

On peut ensuite effectuer une séparation du méthylmercaptan et de l'eau restante, de préférence par décantation, de façon à obtenir :
- un flux (V) comprenant du méthylmercaptan et de l'eau, de préférence à l'état liquide ;
- un flux (W) comprenant de l'eau, de préférence à l'état liquide ; et
- un évent E2.

En particulier, lors de l'étape c4), le flux (W) comprend au moins 50% en poids, de préférence au moins 70%, plus préférentiellement au moins 90% en poids d'eau, par rapport au poids total de l'eau présente dans le flux (T).

Lors de l'étape de séparation c4), il est possible de récupérer la phase gazeuse ainsi séparée des flux (W) et (V), qui sont tous deux à l'état liquide. Ce rejet gazeux est appelé Events E2.

Les évents E2 peuvent être envoyés à l'absorbeur méthanol.

Le flux (V) ou le flux (T) obtenu peut ensuite être séché selon le procédé de séchage tel que décrit ci-dessous.

### Etape c5) - Séchage :

Selon un mode de réalisation, le flux (V) obtenu suite aux étapes c1) à c4) est séché selon le procédé de séchage c5). Selon un autre mode de réalisation, le procédé de séchage c5) est effectué sur n'importe quel flux comprenant du méthylmercaptan et de l'eau.

Ledit procédé de séchage c5) du méthylmercaptan comprend les étapes suivantes :
1') on introduit un flux (A) comprenant du méthylmercaptan et de l'eau dans une colonne de distillation (1) ;
2') on distille ledit flux (A) dans ladite colonne (1) ;
3') on récupère le distillat (B) à l'état gazeux, de préférence en tête de colonne ;
4') on condense le distillat (B), de préférence dans un condenseur (2), de façon à obtenir un condensat (C) à l'état liquide ;
5') on sépare, de préférence à l'aide d'un décanteur (3), ledit condensat (C) de façon à obtenir deux phases liquides séparées :
   - une phase aqueuse (D) ; et
   - une phase organique (E) comprenant du méthylmercaptan ;
6') éventuellement on introduit en tout ou partie la phase organique (E) dans la colonne de distillation (1) en tant que reflux ; et
7') éventuellement on récupère un flux (F) comprenant le méthylmercaptan séché, de préférence en fond de la colonne (1) ; et
8') on récupère l'évent E3 de ladite phase aqueuse (D), de préférence après décantation.

En particulier, on entend par « méthylmercaptan séché » un méthylmercaptan comprenant entre 0 et 1500 ppm, de préférence entre 0 et 1000 ppm, par exemple entre 10 et 800 ppm, plus préférentiellement entre 40 et 800 ppm d'eau, par rapport au total en poids du méthylmercaptan et de l'eau. Il est récupéré de la colonne de distillation, de préférence en fond de colonne de distillation.

La distillation de l'étape 2') peut être effectuée à une pression comprise entre 0,05 et 75 bars absolus, de préférence entre 1 et 30 bars absolus, plus préférentiellement entre 5 et 15 bars absolus, par exemple à environ 10, 11, 12, 13, 14 ou 15 bars absolus.

La distillation de l'étape 2') peut être effectuée à une température comprise entre 20°C et 200°C, de préférence entre 60°C et 100°C, plus préférentiellement entre 65°C et 95°C ; par exemple entre 70°C et 90°C. De façon préférée, la distillation de l'étape 2) peut être effectuée à une température comprise entre 40°C et 200°C, de préférence entre 80°C et 100°C en fond de colonne, et entre 20°C et 100°C, de préférence entre 60°C et 80°C en tête de colonne.

De façon particulièrement préférée, la distillation de l'étape 2') est effectuée à une pression comprise entre 5 et 15 bars absolus et à une température comprise entre 60°C et 100°C. En particulier, la distillation de l'étape 2') est effectuée à une pression comprise entre 5 et 15 bars absolus et à une température comprise entre 70°C et 90°C. En particulier, la distillation de l'étape 2') est une distillation azéotropique.

La distillation de l'étape 2') peut être réalisée dans n'importe quel type de colonne à distiller connue. Il peut s'agir d'une colonne à plateaux (par exemple plateaux à calottes, plateaux à soupapes ou plateaux perforés) ou à garnissage (par exemple à garnissage en vrac ou structuré). La distillation de l'étape 2') peut être réalisée dans une colonne à plateaux, de préférence comprenant entre 5 et 50 plateaux, plus préférentiellement entre 10 et 40 plateaux, par exemple entre 25 et 30 plateaux. La distillation de l'étape 2') peut également être effectuée dans une colonne à cloison (appelée DWC en langue anglaise pour Divided Wall Column). La cloison peut être fixe ou mobile, par exemple avec un garnissage structuré ou vrac.

Le flux (A) est de préférence à l'état liquide ou gazeux.

De préférence, le flux (A) comprend, voire est constitué de, méthylmercaptan, d'eau et éventuellement de traces de méthanol, d'H₂S et de sous-produits soufrés.

Le flux (A) peut comprendre au moins 90%, de préférence au moins 95%, plus préférentiellement au moins 98%, par exemple au moins 99% en poids de méthylmercaptan, par rapport au total en poids du méthylmercaptan et de l'eau.

Le flux (A) peut comprendre au moins 0,15% en poids d'eau, de préférence au moins strictement supérieur à 0,15% en poids d'eau, par rapport au poids total de l'eau et du méthylmercaptan. Le flux (A) peut comprendre au maximum 30%, de préférence au maximum 10% en poids d'eau, par rapport au total en poids du méthylmercaptan et de l'eau. Le flux (A) peut comprendre entre 0,15%, de préférence strictement supérieur à 0,15%, et 30% en poids d'eau par rapport au total en poids du méthylmercaptan et de l'eau. Le flux (A) peut comprendre entre 0,15%, de préférence strictement supérieur à 0,15%, et 10% en poids d'eau par rapport au total en poids du méthylmercaptan et de l'eau.

De préférence, le flux (A) comprend entre 0,15%, de préférence strictement supérieur à 0,15%, et 5% en poids d'eau par rapport au total en poids du méthylmercaptan et de l'eau.

Par exemple, le flux (A) comprend entre 0,15%, de préférence strictement supérieur à 0,15% et 2%, par exemple entre 0,15% et 1,5% ou entre 0,15% et 1% en poids d'eau, par rapport au total en poids du méthylmercaptan et de l'eau ; le reste pouvant être du méthylmercaptan.

Suite à l'étape de distillation 2') du flux (A), on obtient un distillat (B) gazeux. Ce distillat (B) correspond notamment à un mélange azéotrope, de préférence hétéroazéotrope, en particulier dans les conditions de pression et/ou de température de l'étape de distillation 2').

Ainsi, la distillation de l'étape 2') permet notamment de former un mélange azéotrope (soit une distillation azéotropique). Une fois récupéré et condensé à l'état liquide (condensat (C)), il se retrouve sous forme biphasique, dont les deux phases peuvent être facilement séparées, notamment par décantation.

L'étape de condensation 4') du distillat (B) peut être réalisée par toute technique classique. La condensation peut être réalisée dans un condenseur séparé de la colonne de distillation ou qui peut être intégré à ladite colonne. On obtient alors un condensat (C) à l'état liquide, de préférence comprenant deux phases dont l'une est aqueuse et l'autre organique (et comprenant le méthylmercaptan). Lors de l'étape de condensation 4'), la température peut être comprise entre 20°C et 50°C et/ou la pression peut être comprise entre 5 et 15 bars absolus.

Le distillat (B) et le condensat (C) ont de préférence la même composition.

Lors de l'étape 5') de séparation, toute méthode connue peut être utilisée. On utilise de façon tout à fait préférée la décantation. Lors de l'étape de séparation, la température peut être comprise entre 20°C et 50°C et/ou la pression peut être comprise entre 5 et 15 bars absolus. A l'issue de l'étape 5'), on obtient deux phases liquides séparées :
- une phase aqueuse (D) ; et
- une phase organique (E) comprenant du méthylmercaptan.

Selon un mode de réalisation, la phase aqueuse (D) comprend :
- de l'eau,
- de l'H₂S, de préférence à l'état de traces,
- éventuellement du méthylmercaptan, de préférence à l'état de traces ; et
- éventuellement des sous-produits soufrés, de préférence à l'état de traces.

L'H₂S, et éventuellement le méthylmercaptan et les sous-produits soufrés sont de préférence solubilisés dans ladite phase aqueuse. Ils peuvent être séparés de cette phase aqueuse par tout moyen connu et de préférence par un stripping (entrainement), qui peut être un stripping thermique ou par gaz inerte (par exemple par entrainement à l'azote, au méthane, ou au CO₂). On obtient une phase gazeuse qui forme alors des évents appelés évents E3 ci-après. Les évents E3 peuvent être envoyés dans l'absorbeur méthanol.

Selon un mode de réalisation, la phase organique (E) est récupérée à l'issue de l'étape 5') lorsque l'étape 6') de reflux n'est pas effectuée. Selon un autre mode de réalisation, la phase organique (E) est utilisée en tout ou partie en tant que reflux de la colonne de distillation (1).

Lors de l'étape 6'), le taux de reflux peut être compris entre 0 et 0,99, de préférence entre 0 et 0,60. On entend par « taux de reflux » le ratio massique [phase organique (E)/flux (A)].

Le procédé de séchage peut être réalisé en continu ou en batch, de préférence en continu.

Pendant les étapes 1') à 7') du procédé, la pression peut être comprise entre 0,05 et 75 bars absolus, de préférence entre 1 et 30 bars absolus, plus préférentiellement entre 5 et 15 bars absolus, par exemple à environ 10, 11, 12, 13, 14 ou 15 bars absolus.

Du méthanol, de préférence à l'état de traces, peut être compris dans le flux (A) et/ou le distillat (B) et/ou le condensat (C) et/ou la phase aqueuse (D) et/ou le flux (F).

### Etape E) - Extraction gaz-liquide :

L'extraction gaz-liquide peut être effectuée dans au moins une colonne d'absorption ou dans au moins une cuve, de préférence à agitation mécanique. Ladite(lesdites) colonne(s) d'absorption est(sont) notamment choisie(s) parmi les colonnes à garnissage (par exemple garnissage vrac ou structuré), les colonnes à bulles, les colonnes à pulvérisation et les colonnes à film tombant. De préférence, on utilise une(des) colonne(s) à garnissage, par exemple entre 1 et 10 colonnes. Plusieurs colonnes d'absorption peuvent être utilisées, en parallèle ou en série.

Les débits des phases gaz (évents) et liquide (méthanol) dépendent du type et du nombre de colonnes. Les évents gazeux et le méthanol liquide arrivent dans la(les) colonne(s) d'absorption à co-courant ou à contre-courant, de préférence à contre-courant. Par exemple, les évents gazeux arrivent par le bas de la(des) colonne(s) et le méthanol liquide par le haut de la(des) colonne(s).

Ce type de dispositif permettant de réaliser une extraction gaz-liquide est généralement appelé « absorbeur » et dans le cas de la présente invention, « absorbeur au méthanol ».

L'extraction gaz-liquide peut être réalisée à une température comprise entre 0°C et 80°C, par exemple entre 5°C et 80°C, de préférence entre 10°C et 80°C, plus préférentiellement entre 20°C et 70°C. L'extraction gaz-liquide est réalisée à une pression comprise entre 4 et 60 bars absolus, de préférence entre 10 et 50 bars absolus.

Le ratio massique des évents gazeux par rapport au méthanol peut être compris entre 0,001 et 0,5, de préférence entre 0,005 et 0,1.

L'étape E) permet notamment de faire passer les composés soufrés dans le méthanol liquide et de diminuer, voire d'éviter, les rejets de SO₂ dans l'atmosphère.

Les évents gazeux ainsi traités (c'est-à-dire dont au moins un composé soufré a été absorbé dans le méthanol) peuvent être ensuite récupérés, éventuellement incinérés, et relargués dans l'atmosphère avec une teneur en SO₂ diminuée, de préférence lesdits évents ne comprennent quasiment pas ou pas de SO₂.

### Etape F) - Recyclage :

De préférence, le méthanol enrichi ainsi obtenu est utilisé en tant que réactif pour la réaction de l'étape A), éventuellement en mélange avec du méthanol frais.

Par méthanol frais, on entend un méthanol non enrichi au sens de la présente invention, soit un méthanol n'ayant pas subi l'extraction gaz-liquide telle que selon l'invention.

La présente invention concerne également un procédé de traitement des rejets gazeux émis par une unité de production de méthylmercaptan à partir de méthanol et d'H₂S comprenant les étapes suivantes :
- récupération des évents gazeux issus d'au moins une étape de purification du méthylmercaptan, lesdits évents gazeux comprenant au moins un composé soufré, de préférence l'H₂S ;
- réalisation d'une extraction gaz-liquide dudit au moins un composé soufré, de préférence l'H₂S, par du méthanol liquide de façon à obtenir un méthanol liquide enrichi en composé(s) soufré(s), de préférence en H₂S ; et
- éventuellement utilisation dudit méthanol enrichi en tant que réactif pour la réaction de production de méthylmercaptan à partir de méthanol et d'H₂S.

L'ensemble des éléments du procédé de traitement des rejets gazeux (notamment la réaction de production de méthylmercaptan, ladite au moins une étape de purification, lesdits évents gazeux, ledit au moins un composé soufré et ladite extraction gaz-liquide) sont tels que définis pour le procédé de production de méthymercaptan tel que selon l'invention.

### Description des Figures

**Figure** 1 :

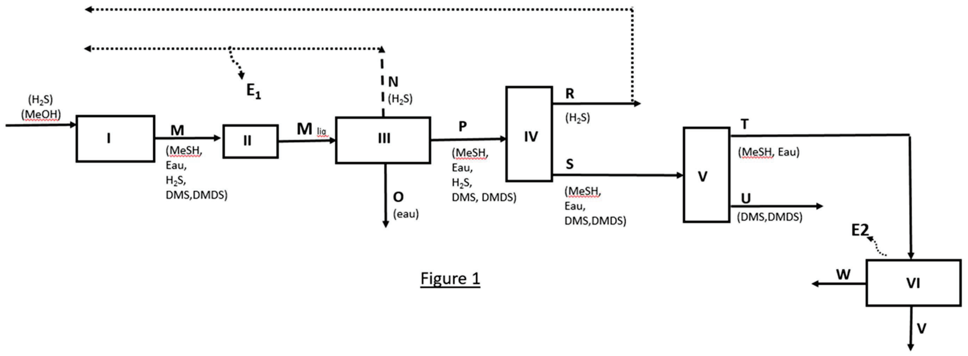

   La Figure 1 représente un mode de réalisation d'un procédé de production de méthylmercaptan par voie méthanol d'où sont récupérés les évents E1 et E2. L'étape A) de réaction est réalisée dans un réacteur (I) à partir de méthanol et d'H₂S. Le flux (M) sortant du réacteur (I) comprend du MeSH, de l'eau, de l'H₂S et des sous-produits soufrés. Le flux (M) est condensé dans un condenseur (II). Il est ensuite séparé dans un décanteur (III) en trois flux :
   - un flux (N) comprenant de l'H₂S,
   - un flux (O) comprenant de l'eau, et
   - un flux (P) comprenant du MeSH, de l'eau, de l'H₂S et des sous-produits soufrés.
   Le flux (N) est purgé et ladite purge représente l'évent E1.
   Le flux (P) est distillé dans une colonne de distillation (IV) pour éliminer l'H₂S (flux (R) en tête de colonne) et obtenir un flux (S) en pied de colonne comprenant du MeSH, de l'eau et des sous-produits soufrés. Le flux (S) est ensuite distillé dans une colonne de distillation (V) pour obtenir un flux (U) en pied de colonne comprenant les sous-produits soufrés et un flux (T) en tête de colonne comprenant le MeSH et l'eau. Le flux (T) est ensuite séparé dans un décanteur (VI) en un flux (V) comprenant le MeSH et l'eau et un flux (W) comprenant de l'eau. L'évent de ce décanteur est récupéré et représente l'évent E2.
**Figure** 2 :

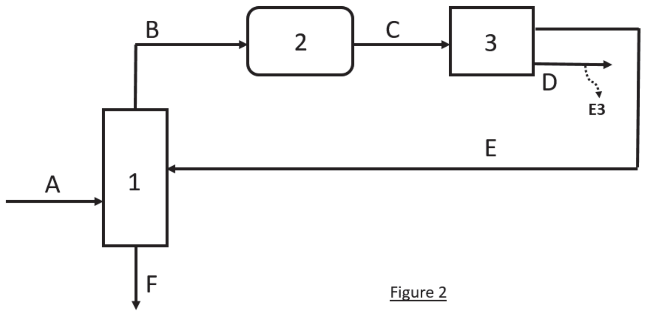

   La Figure 2 représente un mode de réalisation du procédé de séchage dans lequel l'évent E3 est récupéré.
   Le flux (A) entre dans la colonne de distillation (1). Le flux (A) est distillé dans la colonne (1). Le distillat (B) est récupéré en tête de colonne sous forme gazeuse. Le distillat (B) est ensuite condensé dans un condenseur (2) où il est récupéré sous forme liquide biphasique (condensat (C)). Le condensat (C) décante ensuite dans le décanteur (3) afin d'obtenir :
   - une phase aqueuse (D), et
   - une phase organique (E).
   La phase organique (E) sert ensuite de reflux à la colonne de distillation (1). Après décantation, l'évent E3 est récupéré par stripping avec un gaz inerte de la phase aqueuse (D).
   Le méthylmercaptan séché est récupéré en fond de colonne (1) (flux (F)).

### L'expression « compris entre X et X » inclut les bornes mentionnées, sauf mention contraire.

Les exemples qui suivent permettent d'illustrer la présente invention mais ne sont en aucun cas limitatifs.

### EXEMPLES

### Exemple 1 : Exemple comparatif, sans extraction gaz-liquide

Les conditions sont les suivantes :
Dans une unité de production de méthylmercaptan, les évents E1, E2 et E3 ont été récupérés tel que mentionné en Figures 1 et 2.

Après récupération, les évents E1, E2 et E3 sont réunis et leur composition est la suivante :

**[Table 1]**

| Composant | Quantité (% en poids par rapport au poids total des évents) |
|---|---|
| H₂S | 74,5 |
| MesH | 16,9 |
| Inertes | 8,2 |
| Eau | 0,3 |
| Sous-produits soufrés (DMS et DMDS) | 0,1 |
| TOTAL | 100 |

Pour 100 tonnes/jour de méthylmercaptan produit, 3 tonnes/jour de ces évents sont produits et doivent être incinérés.

Ainsi, pour une unité de production de 100 000 tonnes/an de méthylmercaptan, ces évents peuvent représenter jusqu'à 3000 tonnes/an à incinérer. Leur incinération conduit à 5000 tonnes/an de rejets de SO₂.

### Exemple 2 : Exemple conforme à l'invention, avec extraction gaz-liquide

Les évents E1, E2 et E3 sont récupérés de la même façon que pour l'exemple 1 et la composition des trois évents réunis est la même :

**[Table 2]**

| Composant | Quantité (% en poids par rapport au poids total des évents) |
|---|---|
| H₂S | 74,5 |
| MeSH | 16,9 |
| Inertes | 8,2 |
| Eau | 0,3 |
| Sous-produits soufrés (DMS et DMDS) | 0,1 |
| TOTAL | 100 |

Les évents réunis sont envoyés vers un absorbeur méthanol afin de réaliser l'extraction gaz-liquide selon l'invention.

Ladite extraction est réalisée dans une colonne d'absorption à garnissage, le flux gazeux des évents arrivant par le bas de la colonne et le méthanol liquide arrivant par le haut de la colonne à contre-courant.

La température est de 46°C et la pression est de 27 bars absolus.

Le ratio massique des évents gazeux par rapport au méthanol est de 0,05.

Le méthanol enrichi est récupéré en bas de la colonne avec la composition suivante :

**[Table 3]**

| Composant | **Quantité** (% **en poids)** |
|---|---|
| Méthanol | 95,3 |
| H₂S | 3,4 |
| MeSH | 1 |
| Inertes | 0,14 |
| Eau | 0,14 |
| Sous-produits soufrés (DMS et DMDS) | 0,02 |
| TOTAL | 100 |

Cette absorption permet de récupérer plus de 99% de l'H₂S, du MeSH et des sous-produits soufrés qui étaient destinés à l'incinération sans cet absorbeur. Les inertes quant à eux ne sont quasiment pas absorbés par le méthanol.

Ce méthanol enrichi est ensuite mélangé à du méthanol frais (méthanol n'ayant pas subi l'étape d'extraction) pour être envoyé dans le réacteur où est produit le méthylmercaptan à partir de méthanol et d'H₂S.

Pour une unité de production de 100 000 tonnes/an de méthylmercaptan, ce traitement des évents permet de recycler 2 250 tonnes/an d'H₂S et de récupérer 500 tonnes/an de méthylmercaptan supplémentaires.

De plus, les produits soufrés restant dans les évents sont en quantité négligeable : il n'y a plus de rejet de SO₂.

## Revendications

1. Procédé de production de méthylmercaptan comprenant les étapes suivantes :
A) on fait réagir du méthanol avec de l'hydrogène sulfuré pour former un flux (M), de préférence à l'état gazeux, comprenant du méthylmercaptan, de l'H₂S n'ayant pas réagi et éventuellement des sous-produits soufrés ;
B) éventuellement, on condense ledit flux (M) ;
C) on effectue au moins une étape de purification dudit flux (M) pour obtenir un flux enrichi en méthylmercaptan ;
D) on récupère les évents gazeux issus de ladite au moins une étape de purification, lesdits évents gazeux comprenant au moins un composé soufré, de préférence l'H₂S ;
E) on réalise une extraction gaz-liquide dudit au moins un composé soufré, de préférence l'H₂S, par du méthanol liquide de façon à obtenir un méthanol liquide enrichi en composé(s) soufré(s), de préférence en H₂S ; et
F) éventuellement, on utilise ledit méthanol enrichi en tant que réactif pour la réaction de l'étape A).

2. Procédé de production de méthylmercaptan selon la revendication 1, dans lequel lors de l'étape C), on effectue au moins une étape de séparation de phases, de préférence par décantation, et/ou on procède à au moins une purge.

3. Procédé de production de méthylmercaptan selon l'une quelconque des revendications précédentes, dans lequel le ratio massique des évents gazeux par rapport au méthanol lors de l'étape E) est compris entre 0,001 et 0,5, de préférence entre 0,005 et 0,1.

4. Procédé de production de méthylmercaptan selon l'une quelconque des revendications précédentes, dans lequel l'extraction gaz-liquide est réalisée à une température comprise entre 0°C et 80°C, par exemple entre 5°C et 80°C, de préférence entre 10°C et 80°C, plus préférentiellement entre 20°C et 70°C.

5. Procédé de production de méthylmercaptan selon l'une quelconque des revendications précédentes, dans lequel l'extraction gaz-liquide est réalisée à une pression comprise entre 4 et 60 bars absolus, de préférence entre 10 et 50 bars absolus.

6. Procédé de production de méthylmercaptan selon l'une quelconque des revendications précédentes, dans lequel ledit méthanol enrichi est utilisé en tant que réactif pour la réaction de l'étape A) en mélange avec du méthanol frais.

7. Procédé de production de méthylmercaptan selon l'une quelconque des revendications précédentes, dans lequel ledit méthanol enrichi comprend entre 0,1% et 20% en poids d'H₂S, de préférence entre 1% et 10% en poids d'H₂S, plus préférentiellement entre 1% et 5% en poids d'H₂S, par rapport au poids total du méthanol enrichi.

8. Procédé de production de méthylmercaptan selon l'une quelconque des revendications précédentes, dans lequel lesdits évents gazeux comprennent de l'H₂S, du méthylmercaptan, des composés inertes, éventuellement de l'eau et des sous-produits soufrés tels que le diméthylsulfure et le diméthyldisulfure.

9. Procédé de production de méthylmercaptan selon l'une quelconque des revendications précédentes, dans lequel l'extraction gaz-liquide est effectuée dans au moins une colonne d'absorption ou dans au moins une cuve, de préférence à agitation mécanique.
